# EUROPÄISCHE PATENTANMELDUNG

(11) **EP 3 928 714 A1**
(43) Veröffentlichungstag der Anmeldung: **29.12.2021**
(21) Anmeldenummer: 20186266.1
(22) Anmeldetag: 16.07.2020
(51) Int. Cl.: A61B 10/00, B01L 3/00

(54) **VORRICHTUNG ZUR GEWINNUNG EINER RACHENSPÜLPROBE**

(30) Priorität: 22.06.2020 AT 5012720 U
(71) Anmelder: Lead Horizon GmbH, 1060 Wien (AT)
(72) Erfinder: PUTZ, Michael, 1060 Wien (AT); STEININGER, Christoph, 1060 Wien (AT)
(74) Vertreter: Loidl, Manuela Bettina

(57) **Zusammenfassung**

Die Erfindung betrifft einen Kit für eine Probennahme aus dem Mund- und/oder Rachenrau umfassend ein wiederverschließbares Gruppenbehältnis, das das gesamte Material enthält, das zum Durchführen der Probennahme erforderlich ist, enthaltend zumindest die folgenden Elemente: eine Halterungsvorrichtung für ein mit zumindest einer Kamera ausgestattetes mobilen Endgerät, einen Behälter enthaltend eine Spülflüssigkeit, einen Behälter enthaltend ein Transportmedium, ein Röhrchen zum Einführen in den Transportbehälter, und ein Klebesiegel zur Versiegelung des Behälters B und die Verwendung des Kits in einem Verfahren zur Durchführung einer Rachenspülung.

## Beschreibung

### GEBIET DER ERFINDUNG

Die Erfindung betrifft eine Vorrichtung für eine Probennahme aus dem Mund-und/oder Rachenraum.

### HINTERGRUND DER ERFINDUNG

Das Angebot von Tests für Privatpersonen, die eine Selbsttestung ermöglichen, ermöglicht es vielen Menschen, einfach und ohne Arztbesuch Gesundheits-Informationen zu erhalten, ob sie beispielsweise mit bestimmten Viren oder Bakterien infiziert sind, welche Hormonwerte sie haben oder ob sie einen erhöhten Gehalt an Suchtmitteln im Körper haben. Die Qualität der Tests und deren Auswertung sind üblicherweise durchaus von hoher Qualität, insbesondere wenn die Testung als solche in einem Labor oder anderen qualifizierten Einrichtung durchgeführt wird, beispielsweise ein PCR Test zum Nachweis von Viren. Normalerweise ist es im Eigeninteresse der Personen, diese Informationen zu erhalten. Die neueren Entwicklungen im Zusammenhang mit SARS-Cov-2 haben aber gezeigt, dass es auch wichtig sein kann, eine Bestätigung zum Infektionsstatus zu erhalten, insbesondere eine Bestätigung, dass keine Infektion vorliegt. Diese Bestätigung kann wichtig sein, beispielsweise für die Ausübung der beruflichen Tätigkeit, für Reisen oder andere Tätigkeiten. Auch die Präzision und Verlässlichkeit der Tests sind dabei essentiell, allerdings muss stets sichergestellt werden können, dass die Probe und somit das Ergebnis auch tatsächlich und ohne Zweifel der Person zugeordnet werden kann, für die die Bestätigung ausgestellt wird.

Bisher verfügbare Selbsttests haben dazu keinerlei Sicherheitsmaßnahmen, die eine Fälschung durch Probentausch durch die zu testende Privatperson zuverlässig verhindern. Somit kann jede Probe ins Labor geschickt werden, ohne dass garantiert ist, dass die Probe tatsächlich vom Absender stammt und es können dadurch Bestätigungen für Personen ausgestellt werden, die nicht Originatoren des getesteten Probenmaterials sind.

Es besteht daher ein Bedarf, fälschungssichere Selbsttests anzubieten.

### KURZDARSTELLUNG DER ERFINDUNG

Der vorliegenden Erfindung liegt daher die Aufgabe zugrunde, eine Vorrichtung zur Verfügung zu stellen, bei der sichergestellt ist, dass die Probe tatsächlich von derselben Person stammt welche das Probenmaterial an eine Testinstitution schickt und, abhängig von der zugrundeliegenden Fragestellung, auf deren Namen dann das entsprechendes Zertifikat oder die Bestätigung ausgestellt wird.

Diese Aufgabe wird durch die beanspruchte Erfindung und wie hier beschrieben gelöst.

Die vorliegende Erfindung offenbart einen Kit für eine Probennahme aus dem Mund-und/oder Rachenraum, umfassend ein wiederverschließbares Gruppenbehältnis, das das gesamte Material enthält, das zum Durchführen der Probennahme erforderlich ist, umfassend die folgenden Elemente:
(i) eine Halterungsvorrichtung für ein mit zumindest einer Kamera ausgestattetes mobiles Endgerät, beispielsweise ein Mobiltelefon, beispielsweise eine Einkerbung in welche das Mobiltelefon eingesetzt werden kann,
(ii) einen Behälter A enthaltend eine Spülflüssigkeit (eine Spüllösung),
(iii) einen Behälter B enthaltend ein Transportmedium,
(iv) ein Röhrchen R zum Einführen in den Behälter B,
(v) ein Klebesiegel zur Versiegelung des Behälters B,
(vi) optional eine Anleitung zur Durchführung der Probe,
(vii) optional einen Behälter C zum Schutz des Behälters B.

Die Begriffe Kit, Testbox oder Vorrichtung sind hier gleichbedeutend und austauschbar.

In einer bevorzugten Ausführungsform umfasst der Kit einen eindeutigen Identifikationscode, der beispielsweise im Gruppenbehältnis so positioniert ist, dass er durch die Kamerafunktion des mobilen Endgeräts erfasst werden kann. Der Identifikationscode kann sich an beliebiger, für die Kamera zugänglicher Position im Behältnis befinden. Gemäß einer besonderen Ausführungsform kann der Code so ausgerichtet sein, dass er gescannt werden kann, wenn sich das mobile Endgerät in der Halterungsvorrichtung befindet.

In einer weiteren Ausführungsform ist die Halterungsvorrichtung für das mobile Endgerät eine Einkerbung, ein Schlitz, eine ausklappbare Halterung oder eine eingekerbte Ausbuchtung.

In einer weiteren bevorzugten Ausführungsform enthält der Behälter A eine Salzlösung, beispielsweise eine Kochsalzlösung, bevorzugt eine isotonische Kochsalzlösung. Der Behälter B enthält eine Konservierungslösung, beispielsweise eine Pufferlösung, bevorzugt eine phosphatgepufferte Salzlösung.

In einer Ausführungsform weist das Röhrchen R einen Durchmesser auf, der kleiner ist als die Öffnung die Behälters B. Dadurch kann die Probe nach der Rachenspülung einfach und ohne Verluste in den Behälter B eingebracht werden. Das Röhrchen R kann auch trichterförmig ausgebildet sein, mit einem Ende das kleiner ist als die Öffnung die Behälters B. Der Behälter B kann danach wieder dicht verschlossen und mit einem Klebesiegel versehen werden, auf dem ein eindeutiger Identifikationscode angebracht ist

In einer Ausführungsform ist das Gruppenbehältnis wiederverschließbar.

In einer bevorzugten Ausführungsform ist der Identifikationscode im Gruppenbehältnis und auf dem Klebesiegel eine beliebige Ziffern-, Symbol- oder Buchstabenfolge, ein QR Code oder Barcode. Bevorzugt ist der Identifikationscode auf dem Behältnis und auf dem Klebesiegel ident, es können aber auch unterschiedliche Codierungen sein.

Das Gruppenbehältnis kann aus jedem Material sein, das sich für die Verpackung und den Versand eignet, bevorzugt ist es aus Papier, Karton oder Kunststoff.

Das Gruppenbehältnis kann ein-, zwei- oder mehrteilig sein.

In einer besonderen Ausführungsform ist der Kit zweiteilig und weist ein Außenelement und ein Innenelement auf, wobei das Innenelement Einkerbungen oder Aussparungen für die Aufnahme der Behälter A, B, optional C, das Röhrchen R, eine Halterungsvorrichtung für ein Endgerät und optional weitere Einkerbungen oder Aussparungen aufweist, beispielsweise für das Aufstellen des Behälters B, für eine Gebrauchsanleitung oder das Klebesiegel. Gebrauchsanleitung und Klebesiegel können auch auf den Behältern platziert sein.

In einer besonderen Ausführungsform besteht das Außenelement aus Karton, und das Innenelement aus Karton oder Kunststoff. Beide Elemente können einfärbig, mehrfärbig oder mit beliebigen Aufdrucken versehen sein.

Insbesondere ist das Gruppenbehältnis wiederverschließbar, kann einen Adressaufdruck enthalten, und zur Sendung an eine Empfängeradresse geeignet sein, beispielsweise an den Hersteller, Verkäufer, Arzt oder ein Probenlabor.

Die Behälter sind (wieder)verschließbar. Gemäß einer besonderen Ausführungsform weist der Behälter eine Kappe mit Sicherheits-Drehverschluss auf.

Der Kit wie hier beschrieben kann für die Gewinnung von Substanzen verwendet werden welche durch die Rachenspülung isoliert werden können. Beispielsweise für Drogenscreenings, Hormonbestimmungen oder zum Nachweis von Krankheitserregern, beispielsweise Viren oder Bakterien. Diese Krankheitserreger können Viren sein, wie etwa Coronaviren, insbesondere SARS-CoV-2; Influenzaviren, Metapneumoviren, respiratorische Syncytial-Viren (RSV), oder Bakterien, wie Streptokokken, Pneumokokken, und Meningokokken.

Die vorliegende Erfindung umfasst auch die Verwendung des hier beschriebenen Kits in einem Verfahren zur Gewinnung einer Rachenspülprobe einer Person, wobei die Rachenspülung durch die Person mit einer entsprechenden Anwendungssoftware, insbesondere eine Web-Anwendungssoftware, auf einem, mit zumindest einer Kamera ausgestatteten mobilen Endgerät angeleitet und überwacht wird.

Gemäß einer speziellen Ausführungsform erfolgt eine Authentifikation der Person, wobei die Authentifikation mittels Bildaufnahmen von Ausweisdokumenten und der Person selbst erfolgt.

In einer Ausführungsform wird während der Bildaufnahmen der Person diese durch das Anwendungsprogramm aufgefordert, eine Abfolge von vorgegebenen Positionen einzunehmen, im besonderen Fall beruhen die vorgegebenen Positionen auf einer zufälligen Abfolge.

In einer weiteren Ausführungsform werden die vorgegebenen Positionen durch Auswertung von Belichtungsverhältnissen und/oder körperlichen Merkmalen ermittelt.

In einer besonderen Ausführungsform werden die vorgegebenen Positionen auf dem Bildschirm des mobilen Endgeräts insbesondere mittels Avataren, Emojis oder anderen grafischen Symbolen dargestellt.

### KURZE BESCHREIBUNG DER FIGUR

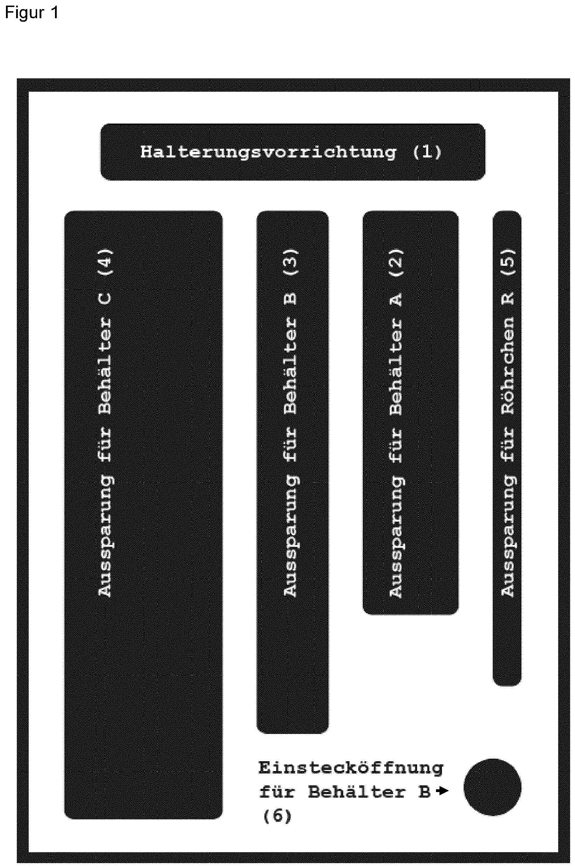

Figur 1 zeigt eine Aufsicht auf eine Ausführungsform des Gruppenbehältnisses im geöffneten Zustand mit Aussparungen für die Halterungsvorrichtung **(1),** den Behälter A **(2),** Behälter B **(3),** Behälter C **(4)** und eine Einstecköffnung für Behälter B **(6).**

### AUSFÜHRLICHE BESCHREIBUNG DER ERFINDUNG

Der Anwender kann über mehrere Kanäle für den privaten oder betrieblichen Gebrauch den Kit erwerben, die alle erforderlichen Materialien enthält, um sicher und nachvollziehbar die Rachenflüssigkeit für mikrobiologische Untersuchungen zu gewinnen, beispielsweise mit Hilfe eines frei zugänglichen Online Web Shops für den privaten Heimgebrauch, oder im Einzelhandel (Drogerie, Apotheke, Lebensmittelhandel, Tankstellen).

Das Gruppenbehältnis kann jede Form haben, die für die Aufnahme der Bestandteile geeignet ist, beispielsweise kann es eine Faltschachtel, eine Stülpdeckel-Schachtel, eine Klappdeckel-Schachtel oder eine Magnetschachtel sein.

Die Abmessungen des Gruppenbehältnisses sind so ausgeführt, dass die angeführten Bestandteile darin aufgenommen werden und verschickt werden können. Beispielsweise kann das Behältnis eine Breite und Länge im Bereich von etwa 80 bis 200 mm haben, beispielsweise von 100 mm bis 180 mm, beispielsweise etwa 110 bis 160 mm. Beispielsweise kann die Länge etwa 160 mm sein, die Breite etwa 110 mm sein. Die Höhe des Behältnisses kann im Bereich von etwa 30 bis 60 mm sein, beispielsweise etwa 40 bis 50 mm.

Das Innenelement wird bevorzugt so ausgestattet, dass die Einzelelemente, die Behälter und das Röhrchen darin fixiert sind und so das Herausfallen oder Durcheinandergeraten während des Transports verhindert wird. Dies kann durch entsprechend dimensionierte Einkerbungen oder Aussparungen erfolgen und/oder durch die Auswahl eines elastischen Materials, wie beispielsweise Kunststoff, Styropor oder Schaumstoff, und so die Elemente zusätzlich fixieren kann. Die Haltevorrichtung für das Endgerät wie hier beschrieben ist bevorzugt so ausgeführt, dass das Endgerät gut fixiert wird und ein Verrutschen während der Rachenspülung verhindert wird. Die Haltevorrichtung kann beliebig ausgeführt sein, beispielsweise eine Einkerbung oder Aussparung, oder eine ausklappbare Vorrichtung an der das Endgerät befestigt, eingespannt oder angelehnt werden kann.

Optional ist eine Einstecköffnung für den Behälter B in aufgestellter Position vorgesehen, wodurch das Einfüllen der Rachenspülung erleichtert wird. Das Einfüllen kann mit dem Röhrchen R erfolgen, das so dimensioniert ist, dass es in den Behälter B eingeführt werden kann, um einen Probenverlust beim Einfüllen zu vermeiden.

Der Identifikationscode kann an jeder beliebigen Stelle auf oder in der Schachtel angebracht sein, solange er mit der Kamerafunktion des Endgeräts erfasst werden kann.

Gemäß einer Ausführungsform ist die Halterungsvorrichtung so ausgeführt, dass die Kamera des Endgeräts direkt auf den Identifikationscode ausgerichtet ist, der sich beispielsweise an der Innenseite des Deckels befindet.

In einer weiteren Ausführungsform ist auf oder in dem Behältnis ein Piktogramm enthalten das den Ablauf der Rachenspülung und aller weiteren Verfahrensschritte erklärt. Das Piktogramm kann beispielsweise auf der Schachtelinnenseite aufgedruckt oder als Anleitung, beispielsweise in Papierform, im Gruppenbehältnis beigelegt sein.

Die Behälter und das Röhrchen können aus jedem geeigneten Material bestehen, bevorzugt sind sie aus Kunststoff, insbesondere aus sterilisierbarem oder bruchsicherem Kunststoff.

Der Behälter A enthält eine Spüllösung, beispielsweise eine Salzlösung. Dies hat den Vorteil, dass für die Isolierung der Substanz kein Nasen- oder Rachenabstrich notwendig ist, sondern die Substanz kann einfach durch Gurgeln und Spülen aus dem Mund- und Rachenraum isoliert werden. Je nachdem, welche Substanz später aus der Rachenspülung isoliert werden soll, kann die Zusammensetzung der Spüllösung variieren. Derartige Lösungen sind der Fachperson bekannt, es kann sich dabei auch um Wasser handeln. Die Menge an Spüllösung kann von der Fachperson bestimmt werden, beispielsweise werden etwa 1 bis 15 ml Spüllösung verwendet, bevorzugt etwa 5 bis 10 ml.

Der Transport der Proben von der Person zum Labor ist ein wichtiges Element in der verlässlichen Testung, insbesondere für den Nachweis von Viren. Die Lagerung von Proben bei zu hohen Temperaturen über lange Zeiträume kann die Verlässlichkeit des Nachweises mittels PCR deutlich reduzieren und damit zu falsch-negativen Befunden führen. Infizierte Personen können sich dadurch in falscher Sicherheit wiegen und weitere Menschen anstecken. Um eine möglichst hohe Verlässlichkeit der Testung zu gewährleisten, werden spezielle Transport- bzw. Konservierungs-medien verwendet, die die Substanzen, beispielsweise Viren, stabilisieren, wodurch die Testung unempfindlicher auf Umwelt-einflüsse gemacht wird.

Der Behälter B enthält eine Koservierungslösung, bevorzugt eine phosphatgepufferte Salzlösung, welche sich beispielsweise für die Aufbewahrung von Viren eignet. Bei anderen Substanzen kann auch hier die am besten geeignete Konservierungslösung gewählt werden.

Der Behälter C ist so dimensioniert, dass der Behälter B zur Gänze aufgenommen werden kann. Optional enthält der Behälter C eine Saugeinlage, die überschüssige oder austretende Flüssigkeiten aufnehmen kann.

Alle Behälter können optional einen Identifikationscode aufweisen.

Das Klebesiegel kann ein Papier- oder Kunststoffsiegel sein, das für einen Einmalgebrauch geeignet ist, wodurch garantiert wird, dass nach Verschließen des Behälters B dieser nicht nochmals geöffnet und das Probenmaterial entfernt oder ausgetauscht werden kann. Somit ist ein zusätzlicher Manipulationsschutz gegeben.

Wenn ein Anwender die Rachenspülung durchführen möchte, kann er das Gruppenbehältnis öffnen und der Anleitung der Gebrauchsanleitung oder des Piktogramms folgen. Alternativ kann mit dem mobilen Endgerät und unter Anwendung eines im Gruppenbehältnis befindlichen Codes wie eines QR- oder Barcodes eine Anleitung für die Mund-/Rachenspülung per Video heruntergeladen und abgespielt werden.

Optional kann ein Formular beigelegt sein, in dem der Anwender die Personendaten ausfüllen und in dem Gruppenbehältnis retournieren kann.

Beispielsweise kann er folgendes Verfahren anwenden, wobei er ein handelsübliches, mit zumindest einer Kamera ausgestattetes mobiles Endgerät benötigt.

Mittels Mobile-Tagging, d.h. durch Auslesen und Decodierung eines eindeutigen Identifikationscodes in der Handlungsanweisung mithilfe der Kamera des mobilen Endgerätes wird der Anwender über den Web-Browser des mobilen Endgerätes direkt zur einer Webanwendung geleitet.

Bei dem Identifikationscode wird vorzugsweise ein zweidimensionaler QR-Code angewendet.

Unter Webanwendung oder Webanwendungssoftware wird dabei ein Anwendungsprogramm nach dem Client-Server-Modell verstanden, bei dem die Datenverarbeitung teilweise auf einem Webserver stattfindet. Die Ergebnisse der Datenverarbeitung werden an den lokalen Client-Rechner des Anwenders, sein mobiles Endgerät, übertragen.

Diese Webanwendung liefert erfindungsgemäß interaktiv detaillierte Handlungsanweisungen. Zuerst wird dabei mittels Erklärvideo der daraufhin folgende Ablauf der Identifizierung erläutert.

Dabei wird der Anwender aufgefordert, vor der Kamera seines mobilen Endgerätes ein Ausweisdokument wie beispielsweise einen Führerschein, Personalausweis oder einen Reisepass zu schwenken und zu bewegen. Die während des Schwenkvorganges erzielten Aufnahmen zeigen das Ausweisdokument aus unterschiedlichen Perspektiven, sodass daraus 3-dimensionale Darstellungen der Dokumente erstellt werden können und die Echtheit mit hoher Sicherheit festgestellt werden kann. Dies insbesondere dann, wenn das Dokument ein Hologramm aufweist.

Danach wird der Benutzer aufgefordert, ein "Selfie"- Video von sich selbst aufzunehmen.

Ein am Display des Handys angezeigter Avatar, ein Emoji oder ein anderes geeignetes grafisches Symbol gibt dem Anwender die nachzuahmende Gestik und einzunehmenden Positionen vor. Durch zufällig ausgewählte Gesten und Emotionen wird verhindert, dass vorgefertigte Videoaufnahmen zum Betrug verwendet werden können.

Die Aufnahmen werden gemeinsam mit den Aufnahmen des Ausweisdokuments dem Identifikationsvorgang zugrunde gelegt.

Nach erfolgreichem Abschluss des Identifikationsvorgangs wird mit einem weiteren Video die Durchführung der Rachenspülung mit der mitgelieferten Spüllösung und den Probengefäßen erläutert.

Damit wird eine verlässliche und hochwertige Probengewinnung gewährleistet.

Um sicherzustellen, dass tatsächlich der identifizierte Benutzer und nicht etwa ein Dritter die Rachenspülung durchführt, wird der Vorgang per Video überwacht.

Weiterhin wird wiederum mittels Mobile-Tagging ein auf dem Probengefäß (Behälter B) aufgebrachter weiterer Identifikations-code eingelesen und dieses Probengefäß dem Anwender zugeordnet.

Der Identifikationscode ist bevorzugt ident mit jenem auf dem Klebesiegel, kann sich alternativ aber auch unterscheiden.

Für den Überwachungsvorgang wird der Anwender angeleitet, sein mobiles Endgerät zum Identifikationscode zu halten oder in einer Halterungsvorrichtung, beispielsweise in einer Testbox oder einem Automaten zu befestigen.

In geeigneter Weise auf einer ebenen Fläche, beispielsweise auf einem Tisch oder einer Platte angeordnet, bringt sie das mobile Endgerät in eine ideale Höhe, Winkel und Position, um einen am Tisch sitzenden Anwender während der Rachenspülung nahtlos im Blick zu behalten und gibt dem Gerät auch die notwendige Stabilität für verwacklungsfreie, störungsfreie Videoaufnahmen. Überdies wird der Anwender dazu angeleitet, sich selbst in den richtigen Abstand zu bringen bis seine Silhouette mit einer am Bildschirm des mobilen Endgerätes angezeigten Umrandung übereinstimmt.

Mittels Timer und akustischer sowie visueller Signalisierung wird dem Anwender die Dauer des Rachenspülvorgangs vorgegeben.

Zur Verhinderung von Manipulationen werden die während des Spülvorganges gemachten Videoaufnahmen mit nicht veränderbaren Zeitmarken versehen.

Der Anwender darf während des Spülvorgangs den Aufnahmebereich der Kamera nicht verlassen, sodass eine lückenlose Kontrolle des Vorgangs möglich ist.

Nach Abschluss des Rachenspülvorgangs wird das Probengefäß mit einem Klebesiegel versiegelt.

Das Klebesiegel weist ebenfalls einen Identifikationscode auf, der mittels Mobile-Tagging erfasst und dem Anwender zugeordnet wird.

Danach ist der Videoüberwachungsprozess abgeschlossen und die Probe kann an einen ausgewählten Empfänger wie beispielsweise direkt ein Labor übersandt werden. Im Labor wird die Unversehrtheit des Klebesiegels überprüft und anhand des Überwachungsvideos die ordnungsgemäße Durchführung der Rachenspülung kontrolliert.

Wenn das der Fall ist, kann eine Gleichstellung zu einer analogen Testung direkt vor einem medizinischen Fachpersonal gewährleistet werden.

Die Erfindung umfasst insbesondere folgende Elemente:
1. Kit für eine Probennahme aus dem Mund- und/oder Rachenraum, umfassend ein wiederverschließbares Gruppenbehältnis, das das gesamte Material enthält, das zum Durchführen der Probennahme erforderlich ist, dadurch gekennzeichnet, dass es die folgenden Elemente umfasst:
   (i) eine Halterungsvorrichtung für ein mit zumindest einer Kamera ausgestattetes mobilen Endgerät, beispielsweise ein Mobiltelefon, beispielsweise eine Einkerbung in welche das Mobiltelefon eingesetzt werden kann,
   (ii) einen Behälter A enthaltend eine Spüllösung,
   (iii) einen Behälter B enthaltend ein Transportmedium,
   (iv) ein Röhrchen R zum Einführen in den Behälter B,
   (v) ein Klebesiegel zur Versiegelung des Behälters B,
   (vi) optional eine Anleitung zur Durchführung der Probe,
   (vii) optional einen Behälter C zum Schutz des Behälters B.
2. Kit nach Element 1, dadurch gekennzeichnet, dass er einen eindeutigen Identifikationscode umfasst, der im Gruppenbehältnis so positioniert ist, dass er durch die Kamerafunktion des mobilen Endgeräts erfasst werden kann.
3. Kit nach Element 1 oder 2, dadurch gekennzeichnet, dass die Halterungsvorrichtung für das mobile Endgerät eine Einkerbung, ein Schlitz, eine ausklappbare Halterung oder eine eingekerbte Ausbuchtung ist.
4. Kit nach einem der Elemente 1 bis 3, dadurch gekennzeichnet, dass der Behälter A eine Salzlösung, bevorzugt eine Kochsalzlösung, bevorzugt eine isotonische Kochsalzlösung enthält.
5. Kit nach einem der Elemente 1 bis 4, dadurch gekennzeichnet, dass der Behälter B eine Pufferlösung enthält, bevorzugt eine phosphatgepufferte Salzlösung.
6. Kit nach einem der Elemente 1 bis 5, dadurch gekennzeichnet, dass das Röhrchen R einen Durchmesser aufweist, der kleiner ist als die Öffnung die Behälters B.
7. Kit nach einem der Elemente 1 bis 6, dadurch gekennzeichnet, dass das Gruppenbehältnis wiederverschließbar ist.
8. Kit nach einem der Elemente 1 bis 7, dadurch gekennzeichnet, dass auf dem Klebesiegel ein eindeutiger Identifikationscode angebracht ist.
9. Kit nach einem der Elemente 1 bis 8, dadurch gekennzeichnet, dass der Identifikationscode eine beliebige Ziffern-, Symbol- oder Buchstabenfolge, ein QR Code oder Barcode ist.
10. Kit nach einem der Elemente 1 bis 9, dadurch gekennzeichnet, dass das Gruppenbehältnis aus Papier, Karton oder Kunststoff besteht.
11. Kit nach einem der Elemente 1 bis 10, wobei das Gruppenbehältnis ein-oder zweiteilig sein kann.
12. Kit nach einem der Elemente 1 bis 11, dadurch gekennzeichnet, dass das Gruppenbehältnis zweiteilig ist und ein Außenelement und ein Innenelement aufweist, wobei das Innenelement Einkerbungen für die Aufnahme der Behälter A, B, optional C, das Röhrchen R, eine Halterungsvorrichtung für ein Mobiltelefon und optional weitere Aussparungen, beispielsweise für eine Gebrauchsanleitung oder das Klebesiegel aufweist.
13. Kit nach einem der Elemente 1 bis 12, dadurch gekennzeichnet, dass das Außenelement aus Karton besteht, und das Innenelement aus Karton oder Kunststoff besteht.
14. Kit nach einem der Elemente 1 bis 13, dadurch gekennzeichnet, dass das Gruppenbehältnis wiederverschließbar ist, optional eine Rücksendeadresse enthält, und zur Sendung an ein Probenlabor geeignet ist.
15. Kit nach einem der Elemente 1 bis 14, dadurch gekennzeichnet, dass die Behälter eine Kappe mit Sicherheits-Drehverschluss aufweisen.
16. Verwendung des Kits nach einem der Elemente 1 bis 15 zur Gewinnung einer Rachenspülung für Drogenscreenings, Hormonbestimmungen oder zum Nachweis von Krankheitserregern, beispielsweise Viren oder Bakterien.
17. Verwendung des Kits nach Element 16, dadurch gekennzeichnet, dass die Krankheitserreger Viren, ausgewählt aus der Gruppe der Coronaviren, insbesondere SARS-CoV-2; Influenzaviren, Metapneumoviren, respiratorische Syncytial-Viren (RSV), oder Bakterien, ausgewählt aus der Gruppe der Streptokokken, Pneumokokken, und Meningokokken sind.
18. Verwendung des Kits nach Element 16 oder 17 in einem Verfahren zur Gewinnung einer Rachenspülprobe einer Person, dadurch gekennzeichnet, dass eine Rachenspülung durch die Person mit einer entsprechenden Web-Anwendungssoftware und einem, mit zumindest einer Kamera ausgestatteten mobilen Endgerät angeleitet und überwacht wird.
19. Verwendung des Kits in einem Verfahren nach Element 18, dadurch gekennzeichnet, dass eine Authentifikation der Person erfolgt und dass diese Authentifikation mittels Bildaufnahmen von Ausweisdokumenten und der Person erfolgt.
20. Verwendung des Kits in einem Verfahren nach Elemente 19, dadurch gekennzeichnet, dass während der Bildaufnahmen der Person diese durch das Anwendungsprogramm aufgefordert wird, eine Abfolge von vorgegebenen Positionen einzunehmen.
21. Verwendung des Kits in einem Verfahren nach Element 20, dadurch gekennzeichnet, dass die vorgegebenen Positionen auf einer zufälligen Abfolge beruhen.
22. Verwendung des Kits in einem Verfahren nach Element 21, dadurch gekennzeichnet, dass die vorgegebenen Positionen durch Auswertung von Belichtungsverhältnissen und/oder körperlichen Merkmalen ermittelt werden.
23. Verwendung des Kits in einem Verfahren nach einem der Elemente 20 bis 22, dadurch gekennzeichnet, dass die vorgegebenen Positionen auf dem Bildschirm des mobilen Endgerätes insbesondere mittels Avataren, Emojis oder anderen grafischen Symbolen dargestellt werden.

## Patentansprüche

1. Kit für eine Probennahme aus dem Mund- und/oder Rachenraum, umfassend ein wiederverschließbares Gruppenbehältnis, das das gesamte Material enthält, das zum Durchführen der Probennahme erforderlich ist, **dadurch gekennzeichnet, dass** es die folgenden Elemente umfasst:
(i) eine Halterungsvorrichtung für ein mit zumindest einer Kamera ausgestattetes mobiles Endgerät, beispielsweise ein Mobiltelefon, beispielsweise eine Einkerbung in welche das Mobiltelefon eingesetzt werden kann;
(ii) einen Behälter A enthaltend eine Spüllösung, bevorzugt eine Kochsalzlösung, besonders bevorzugt eine isotonische Kochsalzlösung;
(iii) einen Behälter B enthaltend ein Transportmedium, bevorzugt eine Pufferlösung, besonders bevorzugt eine phosphatgepufferte Salzlösung;
(iv) ein Röhrchen R zum Einführen in den Behälter B, bevorzugt mit einem Durchmesser, der kleiner ist als die Öffnung des Behälters B;
(v) ein Klebesiegel zur Versiegelung des Behälters B, bevorzugt mit einem eindeutigen Identifikationscode;
(vi) optional eine Anleitung zur Durchführung der Probe;
(vii) optional einen Behälter C zum Schutz des Behälters B.

2. Kit nach Anspruch 1, **dadurch gekennzeichnet, dass** er einen eindeutigen Identifikationscode umfasst, der im Gruppenbehältnis so positioniert ist, dass er durch die Kamerafunktion des mobilen Endgeräts erfasst werden kann.

3. Kit nach Anspruch 1 oder 2, **dadurch gekennzeichnet, dass** die Halterungsvorrichtung für das mobile Endgerät eine Einkerbung, ein Schlitz, eine ausklappbare Halterung oder eine eingekerbte Ausbuchtung ist.

4. Kit nach einem der Ansprüche 1 bis 3, **dadurch gekennzeichnet, dass** der Identifikationscode eine beliebige Ziffern-, Symbol- oder Buchstabenfolge, ein QR Code oder Barcode ist.

5. Kit nach einem der Ansprüche 1 bis 4, **dadurch gekennzeichnet, dass** das Gruppenbehältnis aus Papier, Karton oder Kunststoff besteht, und optional ein- oder zweiteilig ist.

6. Kit nach einem der Ansprüche 1 bis 5, **dadurch gekennzeichnet, dass** das Gruppenbehältnis zweiteilig ist und ein Außenelement und ein Innenelement aufweist, wobei das Innenelement Einkerbungen für die Aufnahme der Behälter A, B, optional C, das Röhrchen R, eine Halterungsvorrichtung für ein Mobiltelefon und optional weitere Aussparungen, beispielsweise für eine Gebrauchsanleitung oder das Klebesiegel aufweist.

7. Kit nach einem der Ansprüche 1 bis 6, **dadurch gekennzeichnet, dass** das Außenelement aus Karton besteht, und das Innenelement aus Karton oder Kunststoff besteht.

8. Kit nach einem der Ansprüche 1 bis 7, **dadurch gekennzeichnet, dass** das Gruppenbehältnis wiederverschließbar ist, optional eine Rücksendeadresse enthält, und zur Sendung an ein Probenlabor geeignet ist.

9. Kit nach einem der Ansprüche 1 bis 8, **dadurch gekennzeichnet, dass** die Behälter eine Kappe mit Sicherheits-Drehverschluss aufweisen.

10. Verwendung des Kits nach einem der Ansprüche 1 bis 9 zur Gewinnung einer Rachenspülung für Drogenscreenings, Hormonbestimmungen oder zum Nachweis von Krankheitserregern, beispielsweise Viren oder Bakterien.

11. Verwendung des Kits nach Anspruch 10, **dadurch gekennzeichnet, dass** die Krankheitserreger Viren, ausgewählt aus der Gruppe der Coronaviren, insbesondere SARS-CoV-2; Influenzaviren, Metapneumoviren, respiratorische Syncytial-Viren (RSV), oder Bakterien, ausgewählt aus der Gruppe der Streptokokken, Pneumokokken, und Meningokokken sind.

12. Verwendung des Kits nach Anspruch 10 oder 11 in einem Verfahren zur Gewinnung einer Rachenspülprobe einer Person, **dadurch gekennzeichnet, dass** eine Rachenspülung durch die Person mit einer entsprechenden Web-Anwendungssoftware und einem, mit zumindest einer Kamera ausgestatteten mobilen Endgerät angeleitet und überwacht wird.

13. Verwendung des Kits in einem Verfahren nach Anspruch 12, **dadurch gekennzeichnet, dass** eine Authentifikation der Person erfolgt und dass diese Authentifikation mittels Bildaufnahmen von Ausweisdokumenten und der Person erfolgt.

14. Verwendung des Kits in einem Verfahren nach Anspruch 13, **dadurch gekennzeichnet, dass** während der Bildaufnahmen der Person diese durch das Anwendungsprogramm aufgefordert wird, eine Abfolge von vorgegebenen Positionen einzunehmen, bevorzugt dass die vorgegebenen Positionen auf einer zufälligen Abfolge beruhen.

15. Verwendung des Kits in einem Verfahren nach Anspruch 14, **dadurch gekennzeichnet, dass** die vorgegebenen Positionen durch Auswertung von Belichtungsverhältnissen und/oder körperlichen Merkmalen ermittelt werden, bevorzugt dass die vorgegebenen Positionen auf dem Bildschirm des mobilen Endgeräts insbesondere mittels Avataren, Emojis oder anderen grafischen Symbolen dargestellt werden.
